# EUROPÄISCHE PATENTSCHRIFT

(11) **EP 0 578 032 B1**
(45) Veröffentlichungstag und Bekanntmachung des Hinweises auf die Patenterteilung: **22.05.1996**
(21) Anmeldenummer: 93109753.9
(22) Anmeldetag: 18.06.1993
(51) Int. Cl.: B01D 21/01, C02F 1/52

(54) **Verfahren zum Betrieb einer Abwasser-Kläranlage und Vorrichtung zum Einspeisen eines Phosphat-Fällmittels**
Sewage treatment plant working process and device for injecting phosphates precipitation products
Procédé de fonctionnement d'une station d'épuration d'eaux usées et dispositif pour l'injection d'un produit de précipitation de phosphates

(30) Priorität: 08.07.1992 DE 4222369
(43) Veröffentlichungstag der Anmeldung: 12.01.1994
(73) Patentinhaber: PROMINENT DOSIERTECHNIK GMBH, D-69123 Heidelberg (DE)
(72) Erfinder: Lauer, Leonhard, Dr., D-6907 Nussloch (DE); Hou, Ranjie, Dr., D-7500 Karlsruhe (DE)
(74) Vertreter: Knoblauch, Ulrich, Dr.-Ing., Patentanwälte Dr. Knoblauch

(56) Entgegenhaltungen:
- EP-A- 0 019 704
- WäGEN + DOSIEREN, Bd.24, Nr.3, Mai 1993, MAINZ, GERMANY Seiten 34 - 36 'Phosphate-Regler mit Fuzzy-Logic'
- KORRESPONDENZ ABWASSER, Bd.35, Nr.4, April 1988, ST.AUGUSTIN,W.GERMANY Seiten 334 - 341 D.GLEISBERG 'Die Fällungsreinigung als bedeutender Bestandteil der weitergehenden Abwasserreinigung'

## Beschreibung

Die Erfindung bezieht sich auf ein Verfahren zum Betrieb einer Abwasser-Kläranlage gemäß dem Oberbegriff des Anspruchs 1 und auf eine Vorrichtung zum Einspeisen eines Phosphat-Fällmittels in eine Abwasser-Kläranlage nach dem Oberbegriff des Anspruchs 7 bzw. 8.

Vielfach besteht die Vorschrift, daß bei öffentlichen Kläranlagen die Phosphatkonzentration im ablaufenden Abwasser unter einem vorgegebenen Grenzwert, beispielsweise 2 mg P/l, liegt. Da dieses Ziel derzeit durch rein biologische Maßnahmen nicht zu erreichen ist, werden dem Abwasser in einer weiteren Reinigungsstufe Fällmittel zugegeben, die, wie Eisen- oder Aluminium-Salze, mit dem Phosphat-Ion schwerlösliche Verbindungen eingehen, die ausfallen und dadurch die Konzentration an gelöstem Phosphor verringern.

Es ist daher bekannt, das Fällmittel durch eine Fällmittel-Pumpe in Abhängigkeit von der Phosphatkonzentration und/oder der Abwassermenge in das Abwasser einzuspeisen. Die Messung der Phosphatkonzentration erfordert jedoch eine Laboruntersuchung oder die Verwendung aufwendiger Analysengeräte, in denen naßchemische Labormethoden automatisch ablaufen. Hierbei ergeben sich nur Einzelsignale, die gegenüber der Probenentnahme um die Analysendauer verzögert sind. Eine einigermaßen genaue Steuerung oder Regelung ist nicht möglich. Vielmehr sind im Hinblick auf die sichere Einhaltung des geforderten Grenzwertes Sicherheits-Überdosierungen notwendig, die neben höheren Betriebskosten zu unerwünscht höheren Metallkonzentrationen im Klärschlamm und zu einem Anstieg des Salzgehaltes im Abwasser führen.

Darüber hinaus sind die automatisch arbeitenden Analysengeräte teuer. Sie benötigen aufgrund ihres photometrischen Auswerteprinzips klare, in der Regel über - wartungsintensive - Ultrafiltration vorgereinigte Proben und müssen von qualifiziertem Personal ständig betreut und mit Chemikalien versorgt werden.

In EP 0 019 704 A1 wird ein gattungsgemäßes Verfahren beschrieben, bei dem dem zulaufenden Abwasser ein Probe entnommen und deren Leitfähigkeit gemessen wird. Durch Zusatz eines Fällmittels wird versucht, das Leitfähigkeitsminimum zu erreichen. Aus dem so gewonnen Fällmittelbedarf wird durch Umrechnung vom Probenvolumen auf das aktuelle Durchfluß-Volumen die tatsächlich benötigte Fällmittelmenge berechnet. Diese Vorgehensweise stellt nicht sicher, daß die Phosphat-Konzentration unter einem vorgegebenen Grenzwert gehalten wird. Darüber hinaus ist die Ermittlung des Leitfähigkeitsminimums zeitaufwendig. In der Zwischenzeit auftretende Schwankungen in der Abwasserbelastung werden nicht erfaßt.

In der gleichen Literaturstelle ist als Stand der Technik angegeben, daß die Bestimmung der Leitfähigkeit des zu behandelnden Wassers zur Steuerung der Dosierung des Fällmittels herangezogen wird. Hierdurch wird aber lediglich die Gesamtverschmutzung des Abwassers, nicht aber seine Phosphatkonzentration berücksichtigt. Denn die Leitfähigkeitsmessung ist die nicht-selektive Summenmessung aller Ionen.

Der Erfindung liegt die Aufgabe zugrunde, eine Abwasser-Kläranlage derart zu betreiben, daß mit geringem Aufwand eine sehr viel bessere Anpassung der Fällmittelmenge an den für die Phosphatausscheidung benötigten Bedarf erreicht wird.

Diese Aufgabe wird verfahrenstechnisch gemäß der Erfindung durch die Merkmale des Anspruchs 1 gelöst.

Dieser Vorschlag beruht auf der überraschenden Feststellung, daß die elektrolytische Leitfähigkeit im zulaufenden Wasser sehr gut mit dessen Phosphatkonzentration korreliert. Dieses Ergebnis kann wegen den geringen Phosphatkonzentrationen und den geringen Äquivalent-Leitfähigkeiten von HPO₄⁻⁻ und H₂PO₄⁻ den genannten Ionen nicht direkt zugeordnet werden, zumal dieser Zusammenhang beim ablaufenden Abwasser nicht mehr vorhanden ist, also im Verlauf der Behandlung innerhalb der Kläranlage verlorengeht. Offenbar führte die "Antiphosphat-Kampagne" bei den Wasch- und Reinigungsmitteln dazu, daß heute die menschlichen und tierischen Ausscheidungen als Phosphatquelle dominieren, und bei diesen Ausscheidungen ist offensichtlich ein Zusammenhang zwischen der Phosphatkonzentration und dem Salzgehalt und damit auch der elektrolytischen Leitfähigkeit denkbar.

Auch wenn die Korrelation von Kläranlage zu Kläranlage unterschiedlich sein mag, bildet die Leitfähigkeit eine On-line-Ersatzgröße, die kontinuierlich und verzögerungsfrei zur Verfügung steht. Die Leitfähigkeitsmessung erfordert einen sehr geringen Aufwand, da entsprechende Meßgeräte gegenüber der Phosphatkonzentrationsmessung geringere Investitions- und Betriebskosten fordern, keiner intensiven Wartung bedürfen und hinsichtlich der Meßwasserqualität keine hohen Anforderungen stellen.

Da sowohl die Menge des durchlaufenden Abwassers als auch die Leitfähigkeit kontinuierlich gemessen werden, erhält man kontinuierlich ein Frachtsignal. Sowohl Änderungen der Durchflußmenge als auch Änderungen der Leitfähigkeit führen zu einer Änderung der einzuspeisenden Fällmittelmenge.

Durch Einzelmessungen der Phosphatkonzentration wird das Frachtsignal korrigiert. Eine einmal vorgenommene Korrektur, beispielsweise durch einen Korrekturfaktor, führt dazu, daß die erzielte Verbesserung auch bei einem sich anschließend ändernden Frachtsignal erhalten bleibt.

Insbesondere kann der Korrekturfaktor mit den Maßnahmen des Anspruchs 2 ermittelt werden. Mit Hilfe dieses Korrekturfaktors, der nicht über den gesamten Meßbereich konstant sein muß, läßt sich das Leitfähigkeitssignal direkt in ein die Phosphatkonzentration kennzeichnendes Signal umwandeln.

Bei einem Vorgehen nach Anspruch 3 berücksichtigt die Korrektur auch die Reduzierung des Fällmittelbedarfs aufgrund der biologischen Phosphataufnahme im Belebungsbecken; eine Reduktion, die sich einer direkten Messung entzieht. Mit Hilfe der Leitfähigkeitsmessung läßt sich dann eine dem jeweiligen Bedarf gut angepaßte Regelung erzielen.

Bei der Alternative nach Anspruch 4 erhält man zwar die genannten Konzentrations-Einzelmessungen mit Verzögerungen (wegen des Durchlaufs durch die Kläranlage und wegen der Analysendauer); das Frachtsignal gibt jedoch die aktuell erforderlichen Änderungen sofort an den Regelkreis weiter.

Mit den Maßnahmen des Anspruchs 5 ergibt sich eine noch bessere Korrelation zwischen elektrolytischer Leitfähigkeit und Phosphatkonzentration.

Mit Vorteil erfolgt die Leitfähigkeitsmessung gemäß Anspruch 6 im Vorklärbecken, also dort, wo das zulaufende Abwasser noch den vollen Phosphatanteil enthält.

Eine Vorrichtung zum Einspeisen eines Phosphat-Fällmittels ist erfindungsgemäß durch die Merkmale des Anspruchs 7 gekennzeichnet.

Eine Alternative ist erfindungsgemäß durch die Merkmale des Anspruchs 8 gekennzeichnet.

Die Erfindung wird nachstehend anhand in der Zeichnung dargestellter Ausführungsbeispiele näher erläutert. Es zeigen in schematischer Darstellung:
- Fig. 1: eine Abwasser-Kläranlage mit einer Pumpensteuerung zur Simultanfällung und
- Fig. 2: eine Abwasser-Kläranlage mit einer Pumpenregelung zur Simultanfällung.

Bei beiden Kläranlagen gibt es ein Vorklärbecken 1, ein Belebungsbecken 2, ein Nachklärbecken 3 und einen Vorfluter 4, die hintereinander geschaltet sind. Ein Zulaufkanal 5 führt zum Vorklärbecken. Verbindungskanäle 6, 7 und 8 verbinden die übrigen Teile der Kläranlage miteinander. Aus dem Nachklärbecken wird über eine Leitung 9 Rücklaufschlamm abgeführt, der teilweise über eine Zweigleitung 10 wieder in den zum Belebungsbecken führenden Verbindungskanal 6 eingespeist wird. Der Rest des Rücklaufschlamms wird mit dem aus dem Vorklärbecken 1 abgezogenen Schlamm als Überschußschlamm über die Leitung 11 abgeführt. Eine Fällmittel-Pumpe 12 fördert über eine Speiseleitung 13 Fällmittel in die Zweigleitung 10, wo sich das Fällmittel mit dem Rücklaufschlamm vermischt, so daß es gut verteilt über den Verbindungskanal 6 in das Belebungsbecken gelangt. Selbstverständlich ist auch eine direkte Dosierung in das Belebungsbecken 2 denkbar. Die Fällmittel-Pumpe 12 besitzt eine Stellvorrichtung 14, mit deren Hilfe die Fördermenge eingestellt wird, und ein von Hand verstellbares Korrekturglied 15, mit welchem die Fördermenge willkürlich geändert werden kann.

Eine Durchflußmengenmeßvorrichtung 16 ist in den Zulaufkanal 5 geschaltet. Der zugehörige Meßwertgeber 17 gibt ein Durchflußmengen-Signal Q an eine Steuervorrichtung 18, die beispielsweise einen Rechner aufweisen kann. Eine Leitfähigkeits-Meßvorrichtung 19 mißt im Vorklärbecken die elektrolytische Leitfähigkeit. Ein zugehöriger Meßwertgeber 20 gibt ein Leitfähigkeitsignal LF an die Steuervorrichtung 18. Diese multipliziert die beiden Werte zu einem Frachtsignal X, das als Stellgröße der Stellvorrichtung 14 zugeführt wird. Dem Nachklärbecken 3 folgt eine Probeentnahmestelle 21, die einem Analysegerät 22 zugeordnet ist, mit dessen Hilfe die verbleibende Phosphatkonzentration angezeigt werden kann. Liegt der angezeigte Wert oberhalb des zulässigen Grenzwertes, wird mit Hilfe des Korrekturgliedes 14 die geförderte Fällmittelmenge erhöht. Dies entspricht einem Korrekturfaktor des Frachtsignals X. Diese Korrektur bleibt proportional erhalten, auch wenn sich das Frachtsignal X ändert.

Selbstverständlich kann ein solches Korrekturglied auch an der Steuervorrichtung 18 vorgesehen sein. Des weiteren kann die Steuervorrichtung 18 auch weitere Faktoren berücksichtigen, wie pH-Wert, Temperatur oder durch die Grund-Leitfähigkeit des Frischwassers hervorgerufenen Offset. Aus diesem Grund sind noch Zusatzeingänge 23 eingezeichnet.

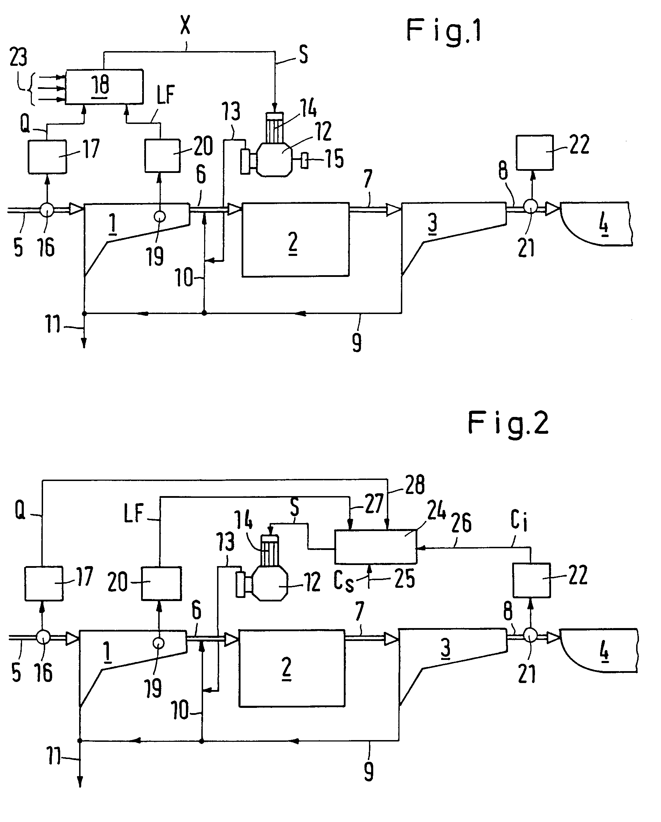

Bei der Ausführungsform der Figur 2 ist eine Regelvorrichtung 24, vorzugsweise in der Form eines Rechners, vorgesehen, die einen Eingang 25 für den Sollwert der Phosphatkonzentration Cₛ im Abwasser besitzt. Über einen Eingang 26 wird von Zeit zu Zeit der Istwert Cᵢ der Phosphatkonzentration vorgegeben. In Abhängigkeit von der Regelabweichung ergibt sich eine Stellgröße S, welche die Stellvorrichtung 14 der Pumpe 12 betätigt. Außerdem werden aber dem Regler noch über einen Eingang 27 das Leitfähigkeitssignal LF und über einen Eingang 28 das Durchflußmengensignal Q zugeleitet. Diese den aktuellen Zustand des Abwassers beschreibenden Werte werden der Regelung als Störgrößen aufgeschaltet, so daß die jeweils eingespeiste Fällmittelmenge mit sehr großer Annäherung dem tatsächlichen Bedarf entspricht.

Die Pumpe 12 kann eine Kolben- oder Membranpumpe mit verstellbarer Frequenz und/oder verstellbarem Hub oder eine beliebige andere Verstellpumpe sein.

Auch die Regelvorrichtung 24 kann mit Zusatzeingängen ähnlich den Eingängen 23 versehen sein. Die Erfindung ist nicht nur bei der Simultanfällung anwendbar, bei der das Phosphat gleichzeitig biologisch und chemisch reduziert wird, vielmehr kann die chemische Fällung auch schon vor der biologischen Reduzierung, also im Vorklärbecken 1 oder Sandfang erfolgen.

## Patentansprüche

1. Verfahren zum Betrieb einer Abwasser-Kläranlage, bei dem dem Abwasser ein Fällmittel für Phosphat zugesetzt wird, wobei die elektrolytische Leitfähigkeit des zulaufenden Abwassers sowie die Menge des durchlaufenden Abwassers kontinuierlich gemessen und multiplikativ mit einer aus der Leitfähigkeit abgeleiteten Größe, die mit der Phosphatkonzentration des zulaufenden Abwassers korreliert, zu einem Frachtsignal vereinigt werden, das zur Bildung einer die Menge des zuzusetzenden Fällmittels bestimmenden Stellgröße verwendet wird, wobei Einzelmessungen der Phosphat-Konzentration erfolgen und das Frachtsignal in Abhängigkeit von diesen Einzelmessungen korrigiert wird.

2. Verfahren nach Anspruch 1, dadurch gekennzeichnet, daß zur Ermittlung eines Korrekturfaktors die Phosphatkonzentration des zulaufenden Abwassers und gleichzeitig dessen Leitfähigkeit gemessen und beide Werte miteinander ins Verhältnis gesetzt werden.

3. Verfahren nach Anspruch 1, dadurch gekennzeichnet, daß der Istwert der Phosphatkonzentration des ablaufenden Abwassers gemessen und eine Korrektur des Frachtsignals im Sinne eines vorgegebenen Sollwerts vorgenommen wird.

4. Verfahren nach einem der Ansprüche 1 bis 3, dadurch gekennzeichnet, daß die Stellgröße durch einen Regler für die Phosphatkonzentration des ablaufenden Abwassers erzeugt wird, dem Einzelmessungen dieser Phosphatkonzentration zugeführt werden und dem das Frachtsignal als Störgröße aufgeschaltet ist.

5. Verfahren nach einem der Ansprüche 1 bis 4, dadurch gekennzeichnet, daß die Leitfähigkeit beeinflussende Faktoren, wie pH-Wert, Temperatur oder Offset, dem Frachtsignal als Störgröße aufgeschaltet werden.

6. Verfahren nach einem der Ansprüche 1 bis 5, dadurch gekennzeichnet, daß die Leitfähigkeitsmessung im Vorklärbecken erfolgt.

7. Vorrichtung zum Einspeisen eines Phosphat-Fällmittels in eine Abwasser-Kläranlage, mit einer Fällmittel-Pumpe (12) und einer die Fördermenge bestimmenden Stellvorrichtung sowie einer Leitfähigkeits-Meßvorrichtung (19) im zulaufenden Abwasser, einer Durchlaufmengen-Meßvorrichtung (16) und einer Steuervorrichtung (18) für die Stellvorrichtung (14), durch gekennzeichnet, daß die Steuervorrichtung (18) aus kontinuierlich gemessenen Leitfähigkeitssignal (LF) und Durchlaufmengensignal (Q) kontinuierlich eine Stellgröße (S) für die Stellvorrichtung (14) bildet, daß im Bereich des ablaufenden Abwassers eine Probenentnahmestelle (21) zur Ermittlung der Phosphatkonzentration in Einzelmessungen angeordnet ist und daß ein Korrekturglied (14) vorgesehen ist, mit dem eine die ermittelte Phosphatkonzentration berücksichtigende Korrekturgröße zur Beeinflussung der Stellgröße eingebbar ist.

8. Vorrichtung zum Einspeisen eines Phosphat-Fällmittels in eine Abwasser-Kläranlage, mit einer Fällmittel-Pumpe (12) und einer die Fördermenge bestimmenden Stellvorrichtung (14) sowie einer Leitfähigkeits-Meßvorrichtung (19) im zulaufenden Abwasser und einer Durchlaufmengen-Meßvorrichtung (16), gekennzeichnet durch eine im Bereich des ablaufenden Abwassers angeordnete Probenentnahmestelle (21) zur Ermittlung der Phosphatkonzentration in Einzelmessungen und durch eine Regelvorrichtung (24), die die Stellgröße (S) für die Stellvorrichtung (14) aus der Regelabweichung der Phosphatkonzentration und durch Störgrößenaufschaltung des kontinuierlich gemessenen Leitfähigkeitssignals (LF) und Durchflußmengensignals (Q) bildet.

## Claims

1. Method for operating a sewage treatment plant, in which a precipitating agent for phosphate is added to the sewage, the electrolytic conductivity of the incoming sewage and the amount of the sewage passing through being measured continuously and combined multiplicatively to form a load signal with a quantity which is derived from the conductivity and which correlates with the phosphate concentration of the incoming sewage, which load signal is used to form a manipulated variable which determines the amount of precipitating agent to be added, individual measurements of the phosphate concentration being carried out and the load signal being corrected as a function of said individual measurements.

2. Method according to Claim 1, characterized in that, to determine a correction factor, the phosphate concentration of the incoming sewage and, simultaneously, its conductivity are measured and both values are compared with one another.

3. Method according to Claim 1, characterized in that the actual value of the phosphate concentration of the outgoing sewage is measured and the load signal is corrected to obtain a set desired value.

4. Method according to one of Claims 1 to 3, characterized in that the manipulated value is generated by a controller for the phosphate concentration of the outgoing sewage, to which controller individual measurements of said phosphate concentration are fed and the load signal is applied as influencing variable.

5. Method according to one of Claims 1 to 4, characterized in that the factors, such as pH, temperature or offset, which influence the conductivity are applied to the load signal as influencing variable.

6. Method according to one of Claims 1 to 5, characterized in that the conductivity measurement is carried out in the preliminary clarification tank.

7. Apparatus for injecting a phosphate precipitating agent into a sewage treatment plant, comprising a precipitating agent pump (12) and a regulating device which determines the pumping rate, as well as a conductivity measuring device (19) in the incoming sewage, a throughput-measuring device (16) and a control device (18) for the regulating device (14), characterized in that the control device (18) continuously forms a manipulated quantity (S) for the regulating device (14) from continuously measured conductivity signal (LF) and throughput signal (Q), in that a sampling point (21) is disposed in the region of the outgoing sewage for determining the phosphate concentration in individual measurements, and in that a correcting element (14) is provided with which a correcting quantity which takes account of the phosphate concentration determined can be inputted to influence the manipulated variable.

8. Apparatus far injecting a phosphate precipitating agent into a sewage treatment plant, comprising a precipitating agent Pump (12) and a regulating device which determines the pumping rate, as well as a conductivity measuring device (19) in the incoming sewage and a throughput measuring device (16), characterized by a sampling point (21) disposed in the region of the outgoing sewage for determining the phosphate concentration in individual measurements and by a regulating device (24) which forms the manipulated variable (S) for the regulating device (14) from the system deviation in the phosphate concentration and by applying the influencing variable of the continuously measured conductivity signal (LF) and throughput signal (Q).

## Revendications

1. Procédé pour l'exploitation d'une station d'épuration des eaux usées, dans laquelle on ajoute aux eaux usées un produit de précipitation pour le phosphate, la conductivité électrolytique des eaux usées affluentes ainsi que la quantité des eaux usées en écoulement étant mesurées en continu et sont réunies de façon multiplicative avec une grandeur dérivée de la conductivité et qui est mise en corrélation avec la concentration de phosphate des eaux usées affluentes en un signal de charge qui est utilisé pour former une grandeur variable déterminant la quantité de produit de précipitation à ajouter, moyennant quoi on effectue des mesures individuelles de la concentration de phosphate et l'on corrige le signal de charge en fonction de ces mesures individuelles.

2. Procédé selon la revendication 1, caractérisé en ce que pour déterminer un facteur de correction, on mesure la concentration de phosphate des eaux usées affluentes et leur conductivité et les deux valeurs sont simultanément mises en relation.

3. Procédé selon la revendication 1, caractérisé en ce que l'on mesure la valeur réelle de la concentration en phosphate des eaux usées effluentes et l'on procède à une correction du signal de charge en termes de valeur nominale prédonnée.

4. Procédé selon l'une des revendications 1 à 3, caractérisé en ce que la grandeur variable est générée par un régulateur pour la concentration de phosphate des eaux usées effluentes, régulateur auquel sont amenées les mesures individuelles de cette concentration de phosphate et auquel est émis le signal de charge en tant que grandeur perturbatrice ou grandeur d'influence.

5. Procédé selon l'une des revendications 1 à 4, caractérisé en ce que les facteurs influençant la conductivité tels que la valeur du pH, la température ou l'offset étant incorporés au signal de charge en tant que grandeur d'influence.

6. Procédé selon l'une des revendications 1 à 5, caractérisé en ce que la mesure de la conductivité s'effectue dans des bassins de première décantation.

7. Dispositif pour alimenter un produit de précipitation de phosphate dans une station d'épuration des eaux usées, avec une pompe de produit de précipitation (12) et un dispositif de régulation déterminant le débit ainsi qu'un dispositif de mesure de conductivité (19) dans les eaux usées affluentes, un dispositif de mesure de débit (16) et un dispositif de commande (18) pour le dispositif de régulation (14), caractérisé en ce que le dispositif de commande (18) forme à partir d'un signal de conductivité (LF) mesuré en continu et d'un signal de débit (Q) mesuré en continu, une grandeur de régulation (S) pour le dispositif de régulation (14), en ce que au niveau des eaux usées effluentes est agencé un poste de prélèvement d'échantillons (21) pour déterminer par des mesures individuelles la concentration en phosphate et en ce qu'il est prévu un élément de correction (14) avec lequel on peut introduire une grandeur de correction prenant en compte la concentration de phosphate déterminée pour influencer la grandeur de régulation.

8. Dispositif pour l'alimentation d'un produit de précipitation de phosphate dans une station d'épuration des eaux usées, avec une pompe de produit de précipitation (12) et un dispositif de régulation (14) déterminant le débit ainsi qu'un dispositif de mesure de la conductivité (19) dans les eaux usées effluentes et un dispositif de mesure de débit (16), caractérisé par un poste de prélèvement d'échantillons (21) agencé dans la zone des eaux usées effluentes pour déterminer la concentration en phosphate dans des mesures individuelles et par un dispositif de régulation (24) qui forme la grandeur de régulation (S) pour le dispositif de régulation (14) à partir de la déviation de régulation de la concentration en phosphate et par intervention de la grandeur perturbatrice ou grandeur d'influence du signal de conductivité mesuré en continu (LF) et du signal de débit (Q).
